# EUROPÄISCHE PATENTSCHRIFT

(11) **EP 1 794 105 B1**
(45) Veröffentlichungstag und Bekanntmachung des Hinweises auf die Patenterteilung: **01.04.2009**
(21) Anmeldenummer: 05776655.2
(22) Anmeldetag: 30.08.2005
(51) Int. Cl.: C07C 7/135, F25J 3/02, G01N 30/54, G01N 30/74

(54) **VERFAHREN UND VORRICHTUNG ZUR SPURENANALYTIK VON STICKSTOFFMONOXID IN OLEFINREICHEN GASEN**
METHOD AND DEVICE FOR CARRYING OUT TRACE ANALYSIS OF NITRIC OXIDE IN OLEFIN-RICH GASES
PROCEDE ET DISPOSITIF D'ANALYSE DE TRACES DE MONOXYDE D'AZOTE DANS DES GAZ RICHES EN OLEFINES

(30) Priorität: 29.09.2004 DE 102004047341; 16.11.2004 DE 102004055120
(43) Veröffentlichungstag der Anmeldung: 13.06.2007
(73) Patentinhaber: Linde Aktiengesellschaft, 80807 München (DE)
(72) Erfinder: HÄRING, Heinz-Wolfgang, 81479 München (DE); KAUFER, Rolf, 82140 Olching (DE); NEUMANN, Hartmut, 80797 München (DE); WENNING, Ulrike, 82049 Pullach (DE)
(86) Internationale Anmeldenummer: PCT/EP2005/009351
(87) Internationale Veröffentlichungsnummer: WO 2006/034766

(56) Entgegenhaltungen:
- DE-A1- 10 105 728
- HALLE R. T.: "Potential Hazards of Nitrogen Oxide Compound Accumulations in Cryogenic Ethylene Recovery Facilities" ETHYLENE PRODUCERS CONFERENCE, Bd. 5, 1994, Seiten 120-182, XP002356003 ISSN: 1066-1557 ISBN: 0-8169-0648-3 in der Anmeldung erwähnt
- GRENOBLE DANE C: "Report on activities of the NOx in Ethylene recovery Task Group" SPRING NAT. MEET. CONF. PROC.; 2004 AICHE SPRING NATIONAL MEETING, CONFERENCE PROCEEDINGS; 2004 AICHE SPRING NATIONAL MEETING, CONFERENCE PROCEEDINGS 2004, April 2004 (2004-04), Seiten 653-662, XP008055411
- BAUER H.: "Nicht-kryogene und gefahrlose Entmethanisierung von FCC-Restgasen" BERICHTE AUS TECHNIK UND WISSENSCHAFT, Bd. 72, 1994, Seiten 15-18, XP002356004 ISSN: 0024-3728
- LITTLEWOOD A.B.: 'Gas Chromatography', 1962, ACADEMIC PRESS, NEW YORK * Seiten 1-15 *
- SIEVERS: '255 Nitrogen Chemiluminescence Detector (NCD)TM' INTERNET CITATION, [Online] 01 Januar 2002, Seiten 1 - 2, XP007905810 Gefunden im Internet: <URL:http://www.ingenieria-analitica.com/Ll ocIA1/PDF/SIEVERS/bro/255_NCD.pdf> [gefunden am 2008-10-01]
- GE: 'Application Note: Measurement of Nitric Oxide in Ethylene by Gas Chromatography and Chemiluminescence Detection' INTERNET CITATION, [Online] 01 Januar 2005, Seiten 1 - 2, XP007905811 Gefunden im Internet: <URL:http://www.geinstruments.com/ionics/in dex.cfm?screen=protectedFile&protectedFile= /SearchableFiles/Library/App.Notes/Protecte d/ncd_10_NO_Measurement_in_Ethylene.pdf> [gefunden am 2008-10-01]
- THIND S.: 'The On-Line Detection of Oxides of Nitrogen in Light Hydrocarbon Streams by Modified Chemiluminescence Detection and/or by Dry Colorimetric Detection' INTERNET CITATION, [Online] 01 April 2005, XP007905820 Gefunden im Internet: <URL:http://www.cianalytics.com/On-Line%20D etection%20of%20Oxides%20-2005.pdf> [gefunden am 2008-10-01]

## Beschreibung

Die Erfindung betrifft ein Verfahren zur Spurenanalytik von Stickstoffmonoxid (NO) in olefinreichen Gasen mittels einer Gaschromatographen(GC)-Säulenschaltung und eines Chemilumineszenzdetektors sowie eine Vorrichtung zur Durchführung des Verfahrens.

In den Anlagenteilen zur kryogenen Aufbereitung (Cold-Box) der Stoffströme in Olefinanlagen stellt Stickstoffmonoxid (NO) - selbst beim Eintrag geringster Spuren - ein Sicherheitsrisiko dar. Bei den dort herrschenden tiefen Temperaturen reagiert NO mit dem immer in geringen Mengen vorhandenen Sauerstoff zu NO₂, welches sich anreichert, mit bestimmten Kohlenwasserstoffen (z. B. Dienen) und Ammoniak weiterreagiert und instabile, und daher explosive nitrose Harze und Salze bildet. In der Vergangenheit führten diese Stoffe schon zu schweren Unfällen (z. B. Cold-Box-Explosion am Steamcracker der Shell in Berre, Frankreich, 1990).

Besteht der Verdacht, dass sich NO im Einsatzstoffstrom zur Cold-Box befindet, werden die Anlagen bisher, um eine Explosion aufgrund eingetragener Stickoxide zu vermeiden, nach einer gewissen Laufzeit prophylaktisch abgeschaltet, angewärmt und ausgewaschen, noch bevor sich eine kritische Menge an nitrosen Harzen und Salzen bilden kann. Wegen der durchzuführenden Reinigungsarbeiten und des Produktionsausfalls stellt diese Prozedur einen erheblichen Kostenfaktor dar. Es besteht daher ein großes Interesse daran, über eine Analytik zu verfügen, die es ermöglicht, die NO-Konzentrationen in olefinreichen Gasströmen bis in den unteren ppb-Bereich quantitativ zu bestimmen. Eine solche Technik erlaubt eine genaue Bilanzierung der eingetragenen NO-Menge und damit die Realisierung möglichst langer Reinigungsintervalle.

Eine weitere Möglichkeit, unnötige Anlagenstillstände zu vermeiden, ist es, die Stickoxide aus dem Einsatzstoffstrom zu entfernen, noch bevor sie in die kryogenen Anlagenteile eingetragen werden. Der Erfolg und die Zuverlässigkeit einer NO-Entfernung kann nur mit Hilfe einer empfindlichen und verlässlichen NO-Spurenanalytik überprüft werden. Dies betrifft zum einen die Arbeit zur Entwicklung eines solchen Verfahrens und zum anderen dessen Einsatz in der Praxis. Ohne eine solche Analytik ist ein Verfahren zur NO-Entfernung weder zu entwickeln noch ist es verkäuflich.

In einer Veröffentlichung von 1993 stellt das "NOₓ Analytical Subcommittee of the NOₓ Task Force of the Ethylene Producer's Committee of the AlChE" einige Mindestanforderungen an eine Technik zur NO-Spurenanalytik in olefinreichen Stoffströmen auf. Um sinnvoll für die oben beschriebenen Zwecke eingesetzt werden zu können, soll eine solche Technik eine Nachweisgrenze von weniger als 50ppb besitzen, dabei schnell und darüber hinaus billig sein. Zwei Methoden werden in der Veröffentlichung empfohlen. Beide (entwickelt von Sievers Instruments bzw. Union Carbide) verwenden Gaschromatographen zur Auftrennung der Gaskomponenten und elektro-optische Detektoren zum NO-Nachweis. Die beiden Methoden sind schnell und, mit Nachweisgrenzen unterhalb von ca. 20ppb, auch ausreichend empfindlich, jedoch nicht billig.

Bei der Methode von Sievers Instruments wird die NO-Detektion mittels ozoninduzierter Chemilumineszenz durchgeführt; der GC beinhaltet lediglich eine gepackte GC-Säule (HayeSep T, 80/100 mesh).

Wenn es darum geht, kleinste NO-Konzentrationen (<1 ppm) in Luft nachzuweisen, werden Chemilumineszenzdetektoren (CLD) seit langem eingesetzt. Sie zeigen nur sehr geringe Querempfindlichkeiten gegenüber CH₄ und wenigen Vol% CO. Dagegen führt bereits das Vorhandensein von geringen Mengen an Olefinen zu Störungen, die einen Nachweis von NO-Spuren unmöglich machen. Aufgabe des GC ist es daher, zu verhindern, dass die NO- und die Olefinfraktionen einer Gasprobe zur gleichen Zeit am CLD eintreffen. In der GC-Säule werden die Olefine stärker adsorbiert als die NO-, CH₄- und CO-Faktionen, und daher von diesen getrennt. Da NO, CH₄ und CO sich nur wenig in ihrem Adsorptionsverhalten unterscheiden, verlassen sie die, lediglich aus einer GC-Säule bestehende GC-Säulenschaltung nahezu gleichzeitig und erreichen gemeinsam den CLD.

Noch bevor Olefine aus der GC-Säule austreten und zum Detektor gelangen können, wird die Messung unterbrochen. Um eine schnellere Desorption der Olefine zu erreichen, wird anschließend die GC-Säule auf ca. 120°C aufgeheizt. Mit Stickstoff, der in dieselbe Richtung wie die zu analysierende Gasprobe strömt, werden die Olefine schließlich aus der GC-Säule gespült. Weil die Olefine die ganze GC-Säule durchströmen müssen, dauert dieser Spülvorgang trotz der Temperaturerhöhung relativ lange.

Union Carbide verwendet einen Photoionisationsdetektor (PID) und einen Gaschromatographen, welcher drei gepackte GC-Säulen (Hayesep T, 60/80 mesh) sowie eine Rückspüleinrichtung enthält. Aufgabe der Rückspüleinrichtung ist es, im Anschluss an die Analyse einer Gasprobe, die in der ersten GC-Säule adsorbierten Olefine wieder zu entfernen. Da PID starke Querempfindlichkeiten gegenüber CH₄ und CO aufweisen, genügt es nicht, wie bei der Sievers-Methode, lediglich die Olefine in einer einzigen GC-Säule abzutrennen, vielmehr sind hier drei GC-Säulen notwendig, um die NO-Fraktion ohne Störkomponenten dem Detektor zuführen zu können.

Die Details der Messverfahren sind in der Veröffentlichung nicht gezeist, wurden dem Anmelder dieser Erfindung jedoch später zugänglich gemacht.

Eine weitere Methode zur NO-Spurenanalytik in olefinreichen Gasen wurde ebenfalls von Sievers entwickelt. Sie verwendet eine GC-Säulenschaltung aus zwei gepackten Säulen und einen CLD zum NO-Nachweis. Der GC ist mit einer Rückspüleinrichtung für die erste GC-Säule ausgestattet. Da die GC-Säulenschaltung nur zwei GC-Säulen beinhaltet, kann auf ein kostspieliges 6-Wege-Ventil, wie es bei der 3-SäulenSchaltung von Union Carbide notwendig ist, verzichtet werden.

Auch zu diesem Verfahren liegen keine Druckschriften vor, die die Details zeigen würden.

Allen diesen Methoden ist gemeinsam, dass die Arbeitstemperatur der Gaschromatographen während der Messung konstant gehalten werden muss. Zu diesem Zweck sind die GC-Säulen in temperaturgeregelten Öfen untergebracht; Temperaturgeregelte Kühlvorrichtungen werden aus praktischen Gründen (höherer Preis, schwierigere Handhabung) meist nicht eingesetzt. Weil die Trennschärfe der GC-Säulen mit sinkender Arbeitstemperatur ansteigt, wird angestrebt, die Ofentemperatur auf einem möglichst geringen Wert konstant zu halten. Aus regelungstechnischen Gründen muss dieser Wert um einige Grad größer sein als die Umgebungstemperatur (z. B. Labortemperatur), und liegt daher i. a. zwischen 30 und 40°C.

Aus DE 101 05 728 A1 ist eien GC-Säulenschaltung mit Rückspüleinrichtung bekannt.

Aufgabe der vorliegenden Erfindung ist es daher, ein Verfahren der eingangs genannten Art sowie eine Vorrichtung zur Durchführung des Verfahrens so auszugestalten, dass Stickoxide in olefinreichen Gasen einfacher als bisher nachgewies en werden können. Die Empfindlichkeit des Verfahrens soll dabei zumindest nicht geringer sein als beim Stand der Technik.

Die Aufgabe wird durch ein Verfahren nach Anspruch 1 und durch eine Vorrichtung nach Anspruch 7 gelöst. Die abhängigen Ansprüche betreffen vorteilhafte Aspekte der Erfindung.

Bei dieser Betriebsweise stellt sich die Temperatur der GC-Säulenschaltung auf einen Wert ein, der sich alleine aus den thermischen Gegebenheiten (z. B. Lufttemperatur, Wärmeeinstrahlung) am Aufstellungsort (z. B. Labor oder Messstelle an einer Anlage) ergibt.

Bevorzugt wird die erfindun gsgemäße NO-Spurenanalytik an Orten aufgestellt und betrieben, an denen sich die Temperatur in der GC-Säulenschaltung auf Werte zwischen 0 und 50°C einstellt.

Eine fallende GC-Temperatur führt sowohl zu einer längeren Retentionszeit (Zeit zwischen dem Eintritt der Gasprobe in den GC und dem NO-Signal am Detektor) als auch zu einer längeren Rückspülzeit, jedoch auch zu einer höheren Trennschärfe des GC. Besonders bevorzugt wird die erfindungsgemäße NO-Spurenanalytik daher an Orten aufgestellt und betrieben, an denen sich die Temperatur in der GC-Säulenschaltung auf Werte zwischen 15 und 40°C einstellt.

Bei der Anwendung des erfindungsgemäßen Verfahrens ergibt sich eine Nachweisgrenze für NO, die unterhalb 10ppb, bevorzugt unterhalb 5ppb und besonders bevorzugt unterhalb 2ppb liegt.

Die Erfindung betrifft ferner eine Vorrichtung zur Spurenanalytik von Stickstoffmonoxid in olefinreichen Gasen mittels einer Gaschromatographen(GC)-Säulenschaltung und eines Chemilumineszenzdetektors.

Eine bevorzugte Ausgestaltung der erfindungsgemäßen Vorrichtung sieht vor, die GC-Säulenschaltung zusammen mit den Ventilen und den Gasleitungen in ein Gehäuse einzubauen. Durch diesen kompakten Aufbau wird erreicht, dass die gesamte NO-Spurenanalytik relativ einfach zu transportieren und flexibel einsetzbar ist. Solange sichergestellt ist, dass die Temperatur am Aufstellungsort allenfalls langsam und mit geringer Amplitude schwan kt, muss das Gehäuse nicht über eine Einrichtung zur Konstanthaltung der GC-Arbeitstemperatur verfügen, insbesonde re muss es sich bei dem Gehäuse nicht um einen temperaturgeregelten Ofen oder/und um eine temperaturgeregelte Kühlvorrichtung handeln.

Die GC-Säulenschaltung wird mit gepackten oder mikrogepackten GC-Säulen (z. B. HayeSep T, 60/80 mesh) aufgebaut. Als besonders geeignet für den Einsatz in der erfindungsgemäßen Vorrichtung haben sich mikrogepackte GC-Säulen (z. B. HayeSep T, 60/80 mesh von Supelco) erwiesen.

Die erfindungsgemäße Vorrichtung ist so ausgelegt, dass sich ei ne Nachweisgrenze für NO ergibt, die unterhalb 10ppb, bevorzugt unterhalb 5ppb und besonders bevorzugt unterhalb 2 ppb liegt.

Die erfindungsgemäße NO-Spurenanalytik kann u. a. dazu eingesetzt werden, die Menge des in die Cold-Box einer Olefinanlage durch einen NO-haltigen Stoffstrom eingetragenen NO zu bestimmen. Dazu werden die NO-Konzentration und die Größe des NO-haltigen Stoffstroms gleichzeitig und in kurzen zeitlichen Abständen gemessen. Da man davon ausgehen kann, dass die Stickoxide die Cold-Box in erster Näherung nicht mehr verlassen, stellt die über die gesamte Messzeit gebildete Summe der Produkte aus NO-Konzentration, Größe des NO-haltigen Stoffstroms und der Zeit zwischen zwei Messungen die NO-Menge dar, die sich während der Messzeit in der Cold-Box angereichert hat. Übersteigt dieser Wert eine festgelegte Schwelle, muss die Olefinanlage abgeschaltet und von den explosiven nitrosen Harzen und Salzen, die sich bei tiefen Temperaturen aus NO bilden, gereinigt werden.

Im Folgenden soll die Erfindung anhand eines Ausführungsbeispiels näher erläutert werden:

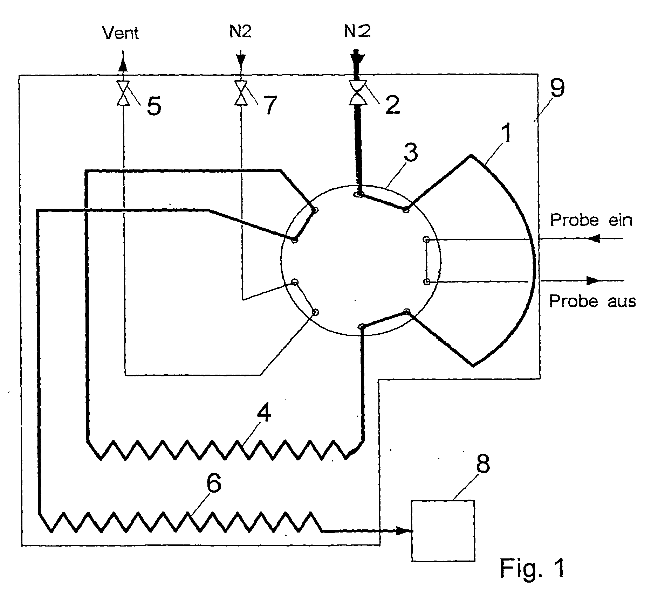

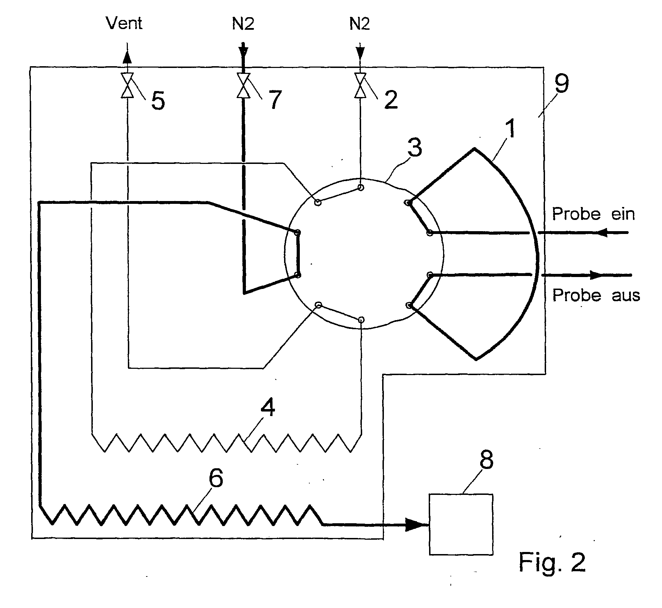

In den Figuren 1 und 2 sind die Stoffströme schematisch dargestellt, wie sie in einer erfindungsgemäßen Apparatur zur Spurenanalytik von NO in olefinreichen Gasströmen mit einer aus zwei GC-Säulen bestehenden GC-Säulenschaltung während der beiden Phasen einer Messung herrschen.

In Figur **1** sind die Verhältnisse während der ersten Phase einer Messung gezeigt: Stickstoff strömt über das Ventil **2** und das 10-Wege-Ventil **3** zur Probesch leife **1,** welche während der vorangegangenen Phase mit einer Gasprobe gefüllt worden ist, und drückt die Gasprobe in die erste GC-Säule **4.** Die Olefine werden dort stärker adsorbiert als NO, CH₄ und CO, welche nach einer gewissen Verweilzeit am anderen Ende der GC-Säule **4** austreten. So bald sich das NO auf der zweiten GC-Säule **6** befindet, wird durch Umschalten des 10-Wege-Ventils **3** die zweite Phase der Messung gestartet.

In Figur 2 sind die Verhältnisse während der zweiten Phase einer Messung - der Spülphase - zu sehen:
Von der Probenahmestelle strömt Gas durch die Probenschleife **1,** füllt diese auf und verlässt anschließend die Apparatur. Die erste GC-Säule **4** wird rückgespült, indem Stickstoff über das Ventil **2** und das 10-Wege-Ventil **3** zur GC-Säule **4** geleitet wird und diese von links nach rechts durchströmt. Dabei werden die Olefine, welche während der vorangegangen Phase adsorbiert wurden, ausgetragen und über das Ventil **5** aus der Apparatur entfernt. Gleichzeitig werden NO, CH₄ und CO, die während der vorangegangenen Phase von den Olefinen getrennten wurden, von Stickstoff, der über die Ventile **7** und **3** einströmt, durch die GC-Säule **6** transportiert, in der eine weitere Auftrennung stattfindet. Schließlich gelangt das NO zum CLD **8** und wird dort detektiert. Nach dem Ende der Rückspülphase wird das 10-Wege-Ventil **3** wieder umgeschaltet und dadurch eine neue Messung gestartet.

Die GC-Säulen, die Ventile und die Gasleitungen sind in einem Gehäuse **9** angeordnet, und somit leicht zu transportieren.

## Patentansprüche

1. Gaschromatographisches Verfahren zur Spurenanalytik von Stickstoffmonoxid (NO) in olefinreichen Gasen mittels eines Chemilumineszenzdetektors (8), mit den Schritten:
Bereitstellen einer Gaschromatographen(GC)-Säulenschaltung aus mehreren gepackten oder mikrogepackten GC-Säulen;
Aufgeben einer gasförmigen Probe auf eine erste Säule (4) der GC-Säulenschaltung;
Rückspülen der ersten Säule (4) der GC-Säulenschaltung, während in einer weiteren Säule (6) ein Teil der aufgegeben Probe weiter zum Chemilumineszenzdetektor (8) transportiert wird;
Erfassen von Stickstoffmonoxidspuren im Austrittsgas der letzten Säule der GC-Säulenschaltung mittels des Chemilumineszenzdetektor (8); und
wiederholtes Durchführen des Probeaufgebens, Rückspülens und Erfassens in kurzen zeitlichen Abständen; **dadurch gekennzeichnet, dass**
das Verfahren ohne Temperaturregelung der GC-Säulenschaltung betrieben wird, wobei sich die Temperatur der GC-Säulenschaltung auf einen Wert einstellt, der sich alleine aus den thermischen Gegebenheiten am Aufstellungsort ergibt.

2. Verfahren nach Anspruch 1, **dadurch gekennzeichnet, dass** die Nachweisgrenze für NO unterhalb 10ppb liegt.

3. Verfahren nach Anspruch 1, **dadurch gekennzeichnet, dass** die Nachweisgrenze für NO unterhalb 5ppb liegt.

4. Verfahren nach Anspruch 1, **dadurch gekennzeichnet, dass** die Nachweisgrenze für NO unterhalb 2ppb liegt.

5. Verfahren nach einem der Ansprüche 1 bis 4, **dadurch gekennzeichnet, dass** die GC-Säulenschaltung an Orten aufgestellt und betrieben wird, an welchen sich die Temperatur der GC-Säulen auf Werte zwischen 0 und 50°C einstellt.

6. Verfahren nach einem der Ansprüche 1 bis 5, **dadurch gekennzeichnet, dass** die GC-Säulenschaltung an Orten aufgestellt und betrieben wird, an welchen sich die Temperatur der GC-Säulen auf Werte zwischen 15 und 40° C einstellt.

7. Vorrichtung ausgestaltet zur Durchführung des gaschromatographisches Verfahren zur Spurenanalytik von Stickstoffmonoxid in olefinreichen Gasen nach einem der Ansprüche 1 bis 6, wobei die Vorrichtung einen Chemilumineszenzdetektor (8) aufweist, wobei die Vorrichtung weiterhin eine Gaschromatographen (GC)-Säulenschaltung aus mehreren gepackten oder mikrogepackten GC-Säulen und eine Einrichtung zur Rückspülung der ersten GC-Säule (4) aufweist, und **dadurch gekennzeichnet, dass** die Vorrichtung nicht mit einer Einrichtung zur Temperaturregelung der GC-Säulenschaltung ausgestattet ist, so dass sich die Temperatur der GC-Säulenschaltung auf einen Wert einstellt, der sich alleine aus den thermischen Gegebenheiten am Aufstellungsort ergibt.

8. Vorrichtung nach einem der Ansprüche 7, **dadurch gekennzeichnet, dass** die GC-Säulenschaltung aus zwei GC-Säulen und einer Einrichtung zur Rückspülung der ersten GC-Säule (4) aufgebaut ist.

9. Vorrichtung nach einem der Ansprüche 7 oder 8, **dadurch gekennzeichnet, dass** die gesamte GC-Säulenschaltung in einem Gehäuse eingebaut ist.

10. Vorrichtung nach einem der Ansprüche 7 bis 9, **dadurch gekennzeichnet, dass** sie so ausgelegt ist, dass die Nachweisgrenze für NO unterhalb 10ppb liegt.

11. Vorrichtung nach einem der Ansprüche 7 bis 9, **dadurch gekennzeichnet, dass** sie so ausgelegt ist, dass die Nachweisgrenze für NO unterhalb 5ppb liegt.

12. Vorrichtung nach einem der Ansprüche 7 bis 9, **dadurch gekennzeichnet, dass** sie so ausgelegt ist, dass die Nachweisgrenze für NO unterhalb 2ppb liegt.

13. Verwendung einer Vorrichtung nach einem der Ansprüche 7 bis 12 in Olefinanlagen, die mit einer Cold-Box ausgestattet sind, und in denen NO-haltige Einsatzstoffströme verarbeitet werden, zur Überwachung der durch die NO-haltigen Einsatzstoffströme in die Cold-Box eingetragenen NO-Menge.

## Claims

1. Gas-chromatographic method of carrying out trace analysis of nitrogen monoxide (NO) in olefin-rich gases by means of a chemiluminescence detector (8), having the steps:
providing a gas chromatograph (GC) column switching system of a plurality of packed or micropacked GC columns;
applying a gaseous sample to a first column (4) of the GC column switching system;
backflushing the first column (4) of the GC column switching system, while in a further column (6) a part of the applied sample is transported further to the chemiluminescence detector (8);
measuring nitrogen monoxide traces in the exit gas of the last column of the GC column switching system by means of the chemiluminescence detector (8); and
repeated carrying out of the sample application, backflushing and measuring in short time intervals; **characterized in that** the method is operated without temperature control of the GC column switching system, wherein the temperature of the GC column switching system adjusts itself to a value which results alone from the thermal conditions at the installation site.

2. Method according to Claim 1, **characterized in that** the detection limit for NO is below 10 ppb.

3. Method according to Claim 1, **characterized in that** the detection limit for NO is below 5 ppb.

4. Method according to Claim 1, **characterized in that** the detection limit for NO is below 2 ppb.

5. Method according to one of Claims 1 to 4, **characterized in that** the GC column switching system is installed and operated at sites at which the temperature of the GC columns adjusts itself to values between 0 and 50°C.

6. Method according to one of Claims 1 to 5, **characterized in that** the GC column switching system is installed and operated at sites at which the temperature of the GC columns adjusts itself to values between 15 and 40°C.

7. Device designed for carrying out the gas-chromatographic method of carrying out trace analysis of nitrogen monoxide in olefin-rich gases according to one of Claims 1 to 6, wherein the device comprises a chemiluminescence detector (8), wherein the device further comprises a gas chromatograph (GC) column switching system of a plurality of packed or micropacked GC columns and an appliance for backflushing the first GC column (4), **characterized in that** the device is not equipped with an appliance for temperature controlling the GC column switching system, and therefore the temperature of the GC column switching system adjusts itself to a value which results alone from the thermal conditions at the installation site.

8. Device according to Claim 7, **characterized in that** the GC column switching system is made up of two GC columns and an appliance for backflushing the first GC column (4).

9. Device according to one of Claims 7 or 8, **characterized in that** the entire GC column switching system is built into a housing.

10. Device according to one of Claims 7 to 9, **characterized in that** it is designed in such a manner that the detection limit for NO is below 10 ppb.

11. Device according to one of Claims 7 to 9, **characterized in that** it is designed in such a manner that the detection limit for NO is below 5 ppb.

12. Device according to one of Claims 7 to 9, **characterized in that** it is designed in such a manner that the detection limit for NO is below 2 ppb.

13. Use of a device according to one of Claims 7 to 12 in olefin plants which are equipped with a cold box and in which NO-containing feedstock streams are processed, for monitoring the NO amount introduced into the cold box by the NO-containing feedstock streams.

## Revendications

1. Procédé de chromatographie gazeuse pour l'analyse de traces de monoxyde d'azote (NO) dans des gaz riches en oléfine au moyen d'un détecteur de chimioluminescence (8), comprenant les étapes suivantes :
élaboration d'un circuit de colonnes de chromatographes gazeux (GC) composé de plusieurs colonnes GC comprimées ou micro-comprimées ;
apport d'un échantillon gazeux dans une première colonne (4) du circuit de colonnes GC ;
lavage par inversion de courant de la première colonne (4) du circuit de colonnes GC, tandis que, dans une autre colonne (6), une partie de l'échantillon apporté est transportée plus loin vers le détecteur de chimioluminescence (8) ;
enregistrement de traces de monoxyde d'azote dans le gaz de sortie de la première colonne du circuit de colonnes GC au moyen du détecteur de chimioluminescence (8) ; et
répétition de l'opération d'apport d'échantillon, de lavage par inversion de courant et d'enregistrement à de brefs intervalles réguliers ; **caractérisé en ce que**
ce procédé est réalisé sans régulation de température du circuit de colonnes GC, la température du circuit de colonnes GC s'établissant à une valeur qui résulte seulement des conditions thermiques sur le lieu de son installation.

2. Procédé selon la revendication 1, **caractérisé en ce que** la limite de détection pour le NO se situe en dessous de 10 ppb.

3. Procédé selon la revendication 1, **caractérisé en ce que** la limite de détection pour le NO se situe en dessous de 5 ppb.

4. Procédé selon la revendication 1, **caractérisé en ce que** la limite de détection pour le NO se situe en dessous de 2 ppb.

5. Procédé selon l'une quelconque des revendications 1 à 4, **caractérisé en ce que** le circuit de colonnes GC est installé et utilisé à des endroits où la température des colonnes GC s'établit à des valeurs comprises entre 0 et 50° C.

6. Procédé selon l'une quelconque des revendications 1 à 5, **caractérisé en ce que** le circuit de colonnes GC est installé et utilisé à des endroits où la température des colonnes GC s'établit à des valeurs comprises entre 15 et 40° C.

7. Dispositif conçu pour la réalisation du procédé de chromatographie gazeuse pour l'analyse de traces de monoxyde d'azote (NO) dans des gaz riches en oléfine selon l'une quelconque des revendications 1 à 6, ce dispositif comportant un détecteur de chimioluminescence (8), ce dispositif comportant en outre un circuit de colonnes de chromatographes gazeux (GC) composé de plusieurs colonnes GC comprimées ou micro-comprimées et d'un dispositif de lavage par inversion de courant de la première colonne GC (4), **caractérisé en ce que** ce dispositif n'est pas équipé d'un système de régulation de température du circuit de colonnes GC, de sorte que la température du circuit de colonnes GC s'établit à une valeur qui résulte seulement des conditions thermiques sur le lieu de son installation.

8. Dispositif selon la revendication 7, **caractérisé en ce que** le circuit de colonnes GC est composé de deux colonnes GC et d'un dispositif de lavage par inversion de courant de la première colonne GC (4).

9. Dispositif selon l'une quelconque des revendications 7 ou 8, **caractérisé en ce que** l'ensemble du circuit de colonnes GC est intégré dans une enceinte.

10. Dispositif selon l'une quelconque des revendications 7 à 9, **caractérisé en ce qu'**il est conçu de manière à ce que la limite de détection pour le NO se situe en dessous de 10 ppb.

11. Dispositif selon l'une quelconque des revendications 7 à 9, **caractérisé en ce qu'**il est conçu de manière à ce que la limite de détection pour le NO se situe en dessous de 5 ppb.

12. Dispositif selon l'une quelconque des revendications 7 à 9, **caractérisé en ce qu'**il est conçu de manière à ce que la limite de détection pour le NO se situe en dessous de 2 ppb.

13. Utilisation d'un dispositif selon l'une quelconque des revendications 7 à 12 dans des installations d'oléfine qui sont équipées d'une boîte froide et dans lesquelles des flux de matière chargée contenant du NO sont traités, pour contrôler la quantité de NO intégrée par l'intermédiaire des flux de matière chargée contenant du NO dans la boîte froide.
